(19) **Europäisches Patentamt European Patent Office Office européen des brevets**

(11) **EP 4 171 511 B1**

(12) **FASCICULE DE BREVET EUROPEEN**

(45) Date de publication et mention de la délivrance du brevet:
**04.09.2024 Bulletin 2024/36**

(21) Numéro de dépôt: **21733161.0**

(22) Date de dépôt: **24.06.2021**

(51) Classification Internationale des Brevets (IPC):
**A61K 9/06** *(2006.01)*       **A61L 27/20** *(2006.01)*
**A61L 27/54** *(2006.01)*

(52) Classification Coopérative des Brevets (CPC):
(C-Sets disponibles)
**A61K 9/06; A61L 27/20; A61L 27/54**       (Cont.)

(86) Numéro de dépôt international:
**PCT/EP2021/067414**

(87) Numéro de publication internationale:
**WO 2021/260145 (30.12.2021 Gazette 2021/52)**

(54) **PROCEDE D'INCORPORATION DE COMPOSES ORGANIQUES EN SOLUTION AU SEIN D'UN HYDROGEL**

VERFAHREN ZUR EINBINDUNG ORGANISCHER VERBINDUNGEN IN LÖSUNG IN EIN HYDROGEL

PROCESS FOR INCORPORATION OF ORGANIC COMPOUNDS IN SOLUTION WITHIN A HYDROGEL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.06.2020 FR 2006622**

(43) Date de publication de la demande:
**03.05.2023 Bulletin 2023/18**

(73) Titulaire: **Laboratoires Vivacy
75116 Paris (FR)**

(72) Inventeurs:
• **TRANCHEPAIN, Frédéric
74350 VILLY LE BOUVERET (FR)**
• **BRUNEL, Florence
74100 ANNEMASSE (FR)**

(74) Mandataire: **Tripoz, Inès
Cabinet Tripoz
Le Pôle Sud
22 rue Seguin
69002 Lyon (FR)**

(56) Documents cités:
**WO-A1-2013/185934       WO-A1-2017/036597**

**US-A1- 2015 232 623**

• KLIMEK KATARZYNA ET AL: "Ion-exchanging dialysis as an effective method for protein entrapment in curdlan hydrogel", MATERIALS SCIENCE AND ENGINEERING C, ELSEVIER SCIENCE S.A, CH, vol. 105, 27 July 2019 (2019-07-27), XP085832158, ISSN: 0928-4931, [retrieved on 20190727], DOI: 10.1016/J.MSEC.2019.110025
• KIM S W ET AL: "Hydrogels: Swelling, Drug Loading and release", ENVIRONMENTAL SCIENCE AND POLLUTION RESEARCH, SPRINGER BERLIN HEIDELBERG, BERLIN/HEIDELBERG, vol. 9, no. 3, 1 January 1992 (1992-01-01), pages 283 - 290, XP002406537, ISSN: 0724-8741, DOI: 10.1023/A:1015887213431

**(Cont. page suivante)**

(52) Classification Coopérative des Brevets (CPC):
(Cont.)

C-Sets
**A61L 27/20, C08L 5/08**

**Description**

L'invention concerne un procédé d'incorporation de composés organiques au sein d'un hydrogel, par exemple un hydrogel à base d'acide hyaluronique, ces composés organiques étant choisis parmi les composés organiques et/ou les oligomères.

Dans la présente demande on désigne par oligomère un polymère comprenant une chaîne constituée d'un petit nombre de motifs monomères, dont le degré de polymérisation (dp) des molécules oligomères varient de deux à quelques dizaines, de préférence dans un intervalle de 2 à 20 ($2 \leq dp \leq 20$).

Dans la suite de la demande ces composés organiques choisis parmi les composés organiques et/ou les oligomères seront désignés par composés organiques.

L'adjonction de composés organiques au sein d'un hydrogel présente un certain nombre de difficultés, dues notamment à la nature physico chimique particulière de l'hydrogel.

Dans le cadre de la présente demande, on appelle « hydrogel » un gel polymérique constitué d'un réseau tridimensionnel constitué d'au moins un polymère, susceptible d'absorber une grande quantité d'eau ou de solution aqueuse et qui présente des propriétés rhéologiques particulières notamment en termes de viscosité et de viscoélasticité.

Ledit réseau peut être constitué par des greffages et/ou des réticulations chimiques par création de liaisons entre les chaines polymériques, ces liaisons pouvant être des liaisons covalentes.

Ledit réseau peut également être obtenu par interactions physiques transitoires par exemple des liaisons ioniques, hydrophobes ou hydrogènes.

En général, l'incorporation de composés organiques qui peuvent être par exemple des principes actifs comme par exemple des anesthésiques, des antioxydants, des vitamines, des acides aminés, des vasoconstricteurs, des vasodilatateurs, des antihémorragiques ou hémostatiques, des antiinflammatoires non stéroïdiens, des antimicrobiens, des hydratants ou régénérants tissulaires ...., est effectuée lors d'une étape dédiée en fin de procédé de préparation du gel lorsque la concentration finale en polymère dans l'hydrogel est atteinte, donc après gonflement, ce qui peut rendre plus complexe ledit procédé, notamment car il implique la plupart du temps une étape d'homogénéisation supplémentaire voire une étape d'ajustement des concentrations.

Or, toute étape d'homogénéisation peut donner lieu à une dégradation plus ou moins importante de l'hydrogel (rupture de liaisons en raison d'une contrainte mécanique) et ainsi altérer les propriétés rhéologiques de l'hydrogel.

Également lors des étapes d'homogénéisation qui peuvent durer plusieurs heures des contaminations microbiennes sont possibles et vont entraîner l'obligation de travailler sous atmosphère contrôlée.

Également des étapes d'homogénéisation qui peuvent durer plusieurs heures vont entraîner une incorporation d'oxygène, qui peut ensuite provoquer des oxydations si le mélange n'est pas effectué sous atmosphère inerte.

De plus, des gradients de concentrations, des différences de solubilité voire des précipitations peuvent être observées car l'homogénéisation de l'hydrogel du fait de ses propriétés rhéologiques et de sa viscosité est difficile.

Une homogénéité parfaite de la concentration en composé organique est difficilement obtenue et l'hydrogel peut être partiellement dégradé.

Le composé organique lui-même peut également entraîner une dégradation potentielle des propriétés rhéologiques et/ou viscoélastiques des formulations ou de leur stabilité, soit directement lors de l'addition, soit lors des phases de stérilisation, soit dans le temps par exemple lors du stockage. La dégradation peut être causée par des concentrations locales excessives, qui entraînent une incompatibilité chimique entre l'hydrogel et le composé organique en concentration locale excessive. Ce dernier cas est un problème important, dans la mesure où une dégradation locale entraînera une injectabilité irrégulière et donc imprévisible.

Le composé organique, notamment lorsqu'il est sous forme salifiée, peut également entraîner une variation de la valeur de pH de la solution de gonflement. Cette variation de pH peut concerner tout l'hydrogel ou une partie seulement ce qui entraînerait des variations de la stabilité en fonction des différents pH.

Dans l'art antérieur, par exemple s'agissant des hydrogels à base d'acide hyaluronique, un certain nombre de démarches ont été entreprises afin d'incorporer un principe actif au sein desdits hydrogels.

Dans la demande WO2005067994 au nom de ANIKA THERAPEUTICS, Inc., il est divulgué que des principes actifs peuvent être dissous dans un solvant de réhydratation de particules sèches d'acide hyaluronique. Cette incorporation ne peut être faite que lorsqu'on dispose de particules d'acide hyaluronique réticulées déshydratées. Il est donc nécessaire d'avoir préalablement effectué une étape de déshydratation, ce qui alourdit le processus industriel.

Dans la demande WO2010052430 au nom de ANTEIS S.A., il est divulgué une composition à base d'acide hyaluronique comprenant un ou plusieurs polyols et de la lidocaïne. Il est déductible du procédé mentionné en exemple 1 que le procédé de fabrication de la composition à base d'acide hyaluronique comprend notamment une étape de dialyse, une étape d'ajout du glycérol, une étape d'ajout du sorbitol, ainsi qu'une étape d'ajout de la lidocaïne. Dans le cadre de cette demande, un grand nombre d'étapes est donc nécessaire, en sus des étapes de préparation de la composition à base d'acide hyaluronique sans autre composé, afin d'ajouter à ladite composition les composés actifs sélectionnés. A ces étapes d'ajout succèdent systématiquement des étapes supplémentaires de mélange et d'homogénéisation, ce qui allonge et complique le procédé.

Dans la demande WO2012104419 au nom de Q-MED AB, il est divulgué l'ajout, dans des compositions à base d'acide hyaluronique, de composés anesthésiants, le composé anesthésiant étant ajouté au moyen d'une « stock solution » (solution hautement concentrée). L'adjonction de l'anesthésiant nécessite donc une étape supplémentaire dans la préparation de la composition à base d'acide hyaluronique. De plus, dans le cas d'un composé peu soluble, préparer une « stock solution » hautement concentrée peut entraîner des difficultés de solubilisation et des problèmes de précipitation.

Dans la demande WO2007128923 au nom de ANTEIS S.A., il est divulgué un procédé de préparation d'un gel biocompatible à libération contrôlée d'un ou plusieurs principes actifs, ainsi que son utilisation dans le domaine thérapeutique et esthétique. Dans le procédé décrit dans cette demande, les principes actifs sont dissous dans un alcool biocompatible ou un mélange d'alcools biocompatibles qui est ensuite ajouté au gel. Ainsi l'incorporation décrite implique une présence obligatoire d'au moins un alcool biocompatible et encore une fois, des étapes d'homogénéisation seront nécessaires.

Dans la demande WO2010015901 au nom d'ALLERGAN, il est divulgué l'adjonction de lidocaïne, cette dernière étant précédée d'un ajustement de pH de la formulation et étant réalisée après gonflement du gel.

Dans les demandes WO2017/036597 et WO2013/185934 au nom de MERZ PHARMA GMBH il est divulgué l'adjonction d'une solution de lidocaïne, cette dernière étant réalisée par incorporation de ladite solution dans les membranes de dialyse et pas dans le bain de dialyse. Ce mode d'incorporation ne peut pas permettre d'équilibrer les concentrations de façon satisfaisante.

En résumé, il existe un besoin de disposer d'un procédé d'incorporation d'un composé organique au sein d'un hydrogel, présentant les avantages/bénéfices suivants :

- adjonction la plus simple possible d'un point de vue processuel ;
- homogénéité de la concentration du composé organique dans l'hydrogel ;
- adjonction en modifiant le moins possible les étapes dudit procédé ;
- absence de nécessité d'homogénéisation de l'hydrogel après l'incorporation du composé organique ;
- pas de préparation de « stock solution » concentrée;
- pas d'ajustement des concentrations à prévoir ;
- etc.

Classiquement lors de la fabrication d'hydrogels une étape de purification est effectuée après toute étape de modification chimique du polymère réticulation ou substitution pour éliminer les réactifs résiduels, les sous-produits de dégradation et toute autre impureté.

L'étape de purification est classiquement réalisée par dialyse de l'hydrogel car les impuretés sont souvent constituées de molécules dont la taille est inférieure à la taille des pores de la membrane de dialyse, ces molécules sont dites « diffusibles ».

La dialyse est basée sur les principes régissant la diffusion à travers une membrane semi-perméable. Les molécules diffusibles vont traverser la membrane selon le gradient de concentration. Il y aura donc un déplacement des molécules du côté le plus concentré vers le côté le moins concentré. Chaque espèce chimique en solution subit individuellement ce processus.

Le but de la dialyse est de réduire la concentration des petites molécules ou molécules diffusibles d'un mélange à un niveau très faible. Les molécules diffusibles vont traverser la membrane du dialysat vers le contre-dialysat. Si on fait une dialyse en continu, on va atteindre une "dilution" infinie, voir par exemple R.F. Boyer (1986) Modem experimental biochemistry, Addison-Wesley Publishing Co, Reading (Mass., USA), p 42-44.

Les membranes sont usuellement sous forme de sacs, de boudins, de poches ... ou tout simplement de membranes semi-perméables.

Les membranes de dialyse sont caractérisées par une limite d'exclusion ("molecular weight cut-off" ou "MWCO") qui est la taille des molécules qui ne pourront pas la traverser (de taille supérieure à la limite) et de celles qui pourront (de taille inférieure à la limite).

À l'équilibre, les concentrations de chaque espèce diffusible seront égales de part et d'autre de la membrane. Si le volume du liquide à l'extérieur de la membrane est très grand par rapport à celui de la solution à dialyser, cette égalité des concentrations implique que la majorité des molécules diffusibles, en terme de quantité (poids), est en fait sortie de la solution. On peut amplifier cette élimination des molécules diffusibles en répétant ce processus.

Il existe deux types de dialyse :

- la dialyse en milieu clos, technique dans laquelle on va laisser aux molécules diffusibles le temps de s'équilibrer de part et d'autre de la membrane et
- la dialyse en continu on expose l'hydrogel à un flux continu de solution de dialyse, en pratique c'est le plus souvent de l'eau ou un tampon pH. Comme le flux est continu, les petites molécules ou molécules diffusibles ne pourront jamais atteindre une concentration d'équilibre et, traverseront ou diffuseront constamment à travers la membrane. On peut ainsi virtuellement atteindre une concentration nulle en ces molécules diffusibles.

Lors d'une dialyse en milieu clos, le diffusat est régulièrement remplacé par une solution de dialyse nouvellement préparée.

Lors d'une dialyse en continu le flux continu est alimenté par une solution de dialyse en continu.

Dans un mode de réalisation concernant les deux types de dialyse, plusieurs solutions de dialyse peuvent être utilisées successivement au cours de la même dialyse. Il s'agira par exemple de solutions de dialyse à des concentrations de composé organique différentes permettant d'incorporer ledit composé organique par paliers successifs de concentrations.

Des techniques voisines comme l'ultrafiltration tangentielle ou l'ultrafiltration sur membranes à fibres creuses peuvent également être utilisées.

La concentration en polymère peut être ajustée par gonflement avant, pendant ou à la suite de cette étape de dialyse dite de purification.

De manière surprenante, il a été mis en évidence par la demanderesse que la technique de dialyse peut être détournée de son objectif de purification et être utilisée pour introduire un composé organique au sein d'un hydrogel, lors d'une étape de dialyse, et plus précisément par l'introduction dudit au moins un composé organique au sein du solvant de dialyse, à savoir comme constituant de la solution de dialyse.

Dans la suite de la description, le liquide utilisé pour alimenter la dialyse sera désigné comme la solution de dialyse ; elle est constituée du solvant de dialyse et des composés en solution.

La solution à l'équilibre dans le gel, à l'intérieur de la membrane sera désignée comme étant le rétentat.

La solution souvent appelée dialysat qui est recueillie à l'extérieur de la membrane sera désignée comme étant le diffusat pour éviter toute confusion.

Par ce procédé la solution de dialyse n'est plus considérée uniquement comme un liquide de lavage ou de dilution mais comme un liquide d'apport de composé organique.

Il a en outre été mis en évidence que par un procédé d'incorporation correspondant à l'invention, le composé organique était réparti de manière parfaitement homogène (sans nécessiter une étape particulière d'homogénéisation), et dans une concentration parfaitement prédictible au maximum égale à la même concentration que dans la solution de dialyse.

Plus généralement, toutes les difficultés générées par l'adjonction d'un composé organique dans un hydrogel, en partie relatées ci-dessus, sont surmontées au moyen de l'invention.

La présente invention concerne un procédé d'incorporation d'au moins un composé organique au sein d'un hydrogel, caractérisé en ce que ladite incorporation est effectuée par dialyse, ledit composé organique étant solubilisé dans la solution de dialyse à une concentration égale ou supérieure à la concentration cible.

Dans un mode de réalisation, ledit composé organique est solubilisé dans la solution de dialyse à une concentration égale à la concentration cible.

Dans un mode de réalisation ledit procédé d'incorporation peut être mis en oeuvre comme étape consécutive et/ou concomitante à une étape d'hydratation dans un procédé de préparation d'un hydrogel.

Dans un mode de réalisation, ledit procédé comprend en outre les au moins étapes de :

a) réticulation en présence d'un réticulant,
b) purification et gonflement.

Dans un mode de réalisation, le procédé de préparation d'un hydrogel comprend en outre après la mise en oeuvre dudit procédé d'incorporation une étape de stérilisation.

Dans un mode de réalisation, le procédé de préparation d'un hydrogel comprend en outre après l'étape de purification et de gonflement une étape de mélange d'au moins deux gels réticulés différents.

Dans un mode de réalisation, le procédé de préparation d'un hydrogel comprend en outre après l'étape de purification et de gonflement une étape d'incorporation par mélange au polymère réticulé d'une solution de polymère non réticulé.

Dans un mode de réalisation ledit procédé d'incorporation peut être mis en oeuvre sur des gels réticulés préalablement déshydratés, après une étape de réhydratation.

La solution de dialyse est de l'eau ou une solution aqueuse qui peut comprendre en outre ses sels minéraux.

Dans un mode de réalisation, la solution de dialyse comprend du chlorure de sodium.

Dans un mode de réalisation, la solution de dialyse comprend un tampon pH.

Dans un mode de réalisation, la solution de dialyse est une solution tampon pH.

Dans un mode de réalisation, la solution de dialyse est une solution tampon pH choisie dans le groupe constitué des tampons phosphates, des tampons citrates, des tampons borates, des tampons lactates, des tampons carbonates, des tampons acétates et de leurs mélanges, lesdits phosphates, citrates, borates, lactates, carbonates ou acétates étant sous forme acide ou sous forme salifiée. Lorsqu'ils sont sous forme salifiée les contre-ions sont de préférence choisis parmi les ions alcalins comme l'ion sodium (Na+), ou encore l'ion potassium (K+) ou l'ion lithium (Li+).

Dans un mode de réalisation, la solution de dialyse est une solution tampon phosphate.

Dans un mode de réalisation, la solution de dialyse est une solution tampon citrate.

Dans un mode de réalisation, la solution de dialyse est une solution tampon borate.

Dans un mode de réalisation, la solution de dialyse est une solution tampon lactate.

Dans un mode de réalisation, la solution de dialyse est une solution polysaline.

Dans un mode de réalisation, la concentration cible en composé organique, à savoir dans le gel après incorporation est comprise entre 0,01 et 500 mg/g.

Dans un mode de réalisation, ladite dialyse est réalisée à une température comprise entre +2°C et 40°C.

Dans un mode de réalisation, ladite dialyse est réalisée à une température comprise entre 5°C et 15°C.

Dans un mode de réalisation, ladite dialyse est mise en oeuvre en utilisant une membrane dont le seuil de coupure (MWCO-Molecular weight cut-off) est compris entre 1 et 200kDa.

Dans un mode de réalisation, le MWCO est compris entre 6 et 50 kDa, ($6 \leq MCWO \leq 50$ kDa).

Dans un mode de réalisation, le MWCO est compris entre 12 et 14 kDa, $12 \leq MCWO \leq 14$ kDa).

Dans un mode de réalisation, ladite dialyse est mise en oeuvre en milieu clos.

Dans un mode de réalisation, ladite dialyse comprend entre 2 et 10 bains successifs de solution de dialyse, chacun des bains ayant un volume compris entre 1 et 50 fois celui de l'hydrogel dans lequel le composé organique est incorporé.

Dans un mode de réalisation, ladite dialyse comprend entre 4 et 6 bains successifs de solution de dialyse, chacun des bains ayant un volume compris entre 8 et 12 fois celui de l'hydrogel dans lequel le composé organique est incorporé.

Dans un mode de réalisation, ladite dialyse est mise en oeuvre en continu par mise en œuvre d'un flux continu de solution de dialyse à travers l'hydrogel et la membrane.

Dans un mode de réalisation, ladite dialyse comprend entre 2 et 10 passes successives de solution de dialyse, chacune des passes ayant un volume compris entre 1 et 400 fois celui de l'hydrogel dans lequel le composé organique est incorporé.

La dialyse est effectuée jusqu'à ce que la concentration en composé organique dans l'hydrogel soit égale, à l'incertitude de mesure de la concentration près, à la concentration cible qui est au maximum celle de la solution de dialyse.

Dans un mode de réalisation, la dialyse est effectuée jusqu'à ce que les compositions qualitatives et quantitatives de la solution de dialyse, du rétentat et du diffusat (dialysat) soient à l'équilibre et donc que la concentration en composé organique dans l'hydrogel soit égale à l'incertitude de mesure de la concentration près, à la concentration de la solution de dialyse.

La composition qualitative et quantitative de l'hydrogel après dialyse est qualifiée de composition cible, les concentrations en les différents constituants sont qualifiées de concentrations cibles.

Dans un mode de réalisation, le procédé est caractérisé en ce que l'au moins un composé organique est choisi dans le groupe constitué des antioxydants.

Les antioxydants outre les propriétés antioxydantes vis-à-vis des constituants de la matrice hydrogel, présentent des effets anti-âge remarquables sur les kératinocytes épidermiques, les cellules endothéliales et les fibroblastes dermiques

Dans un mode de réalisation, l'antioxydant est choisi dans le groupe des polyols.

Dans un mode de réalisation, le polyol est choisi dans le groupe comprenant le glycérol, le sorbitol, le propylène glycol, le xylitol, le mannitol, l'érythritol, le maltitol et le lactitol.

Dans un mode de réalisation, le polyol est choisi dans le groupe constitué par le mannitol, le sorbitol, le maltitol et le glycérol, seul ou en mélange.

Dans un mode de réalisation, le polyol est choisi dans le groupe constitué par le mannitol, le sorbitol et le maltitol, seul ou en mélange.

Dans un mode de réalisation, le polyol est le mannitol.

Dans un mode de réalisation, le polyol est le sorbitol.

Dans un mode de réalisation, le polyol est le maltitol.

Dans un mode de réalisation, le polyol est le glycérol.

Dans un mode de réalisation, le polyol est un mélange de mannitol et de sorbitol.

Dans un mode de réalisation, l'antioxydant est choisi dans le groupe des dérivés de vitamine C.

Dans un mode de réalisation, le dérivé de vitamine C est choisi dans le groupe comprenant l'ascorbyl phosphate de magnésium, l'ascorbyl phosphate de sodium, l'ascorbyl-2-glucoside.

Dans un mode de réalisation, l'antioxydant est choisi dans le groupe des dérivés de vitamine E et des tocophérols.

Dans un mode de réalisation, l'antioxydant est choisi dans le groupe des caroténoïdes et des rétinoïdes et de leurs dérivés.

Dans un mode de réalisation, les caroténoïdes et les rétinoïdes et leurs dérivés sont choisis dans le groupe comprenant le rétinol, l'acide rétinoïque le rétinal, les esters de rétinol et le carotène.

Dans un mode de réalisation, l'antioxydant est choisi dans le groupe des pseudo-tripeptides.

Dans un mode de réalisation, le pseudo-tripeptide est le glutathion.

Dans un mode de réalisation, l'antioxydant est choisi parmi les différentes formes de la coenzyme Q10, l'ubiquinone ou l'ubiquinol.

Dans un mode de réalisation, la concentration massique en antioxydant dans la solution de dialyse est comprise entre 0,01 mg/g et 200 mg/g.

Dans un mode de réalisation, la concentration massique en antioxydant dans la solution de dialyse est comprise entre 0,01 mg/g et 100 mg/g.

Dans un mode de réalisation, la concentration massique en polyol dans la solution de dialyse est comprise entre 0,01 et 100 mg/g.

Dans un mode de réalisation, la concentration massique en polyol dans la solution de dialyse est comprise entre 0,01 mg/g et 50 mg/g.

Dans un mode de réalisation, la concentration massique en polyol dans la solution de dialyse est comprise entre 10 et 40 mg/g.

Dans un mode de réalisation, la concentration massique en polyol dans la solution de dialyse est comprise entre 15 et 30 mg/g.

Dans un mode de réalisation, la concentration massique en polyol dans la solution de dialyse est comprise entre 15 et 25 mg/g.

Dans un mode de réalisation, la concentration massique en polyol dans la solution de dialyse est comprise entre 20 et 40 mg/g.

Dans un mode de réalisation, la concentration massique en polyol dans la solution de dialyse est comprise entre 20 et 30 mg/g.

Dans un mode de réalisation, la concentration massique en polyol dans la solution de dialyse est comprise entre 25 et 35 mg/g.

Dans un mode de réalisation, la concentration massique en polyol dans la solution de dialyse est 35 mg/g.

Dans un mode de réalisation, le polyol est le mannitol et la concentration massique en mannitol dans la solution de dialyse est comprise entre 10 et 40 mg/g.

Dans un mode de réalisation, le polyol est le mannitol et la concentration massique en mannitol dans la solution de dialyse est comprise entre 15 et 30 mg/g.

Dans un mode de réalisation, le polyol est le mannitol et la concentration massique en mannitol dans la solution de dialyse est comprise entre 15 et 25 mg/g.

Dans un mode de réalisation, le polyol est le mannitol et la concentration massique en mannitol dans la solution de dialyse est comprise entre 20 et 40 mg/g.

Dans un mode de réalisation, le polyol est le mannitol et la concentration massique en mannitol dans la solution de dialyse est comprise entre 25 et 35 mg/g.

Dans un mode de réalisation, le polyol est le mannitol et la concentration massique en mannitol dans la solution de dialyse est de 35 mg/g.

Dans un mode de réalisation, le polyol est le sorbitol et la concentration massique en sorbitol dans la solution de dialyse est comprise entre 10 et 40 mg/g.

Dans un mode de réalisation, le polyol est le sorbitol et la concentration massique en sorbitol dans la solution de dialyse est comprise entre 15 et 30 mg/g.

Dans un mode de réalisation, le polyol est le sorbitol et la concentration massique en sorbitol dans la solution de dialyse est comprise entre 15 et 25 mg/g.

Dans un mode de réalisation, le polyol est le sorbitol et la concentration massique en sorbitol dans la solution de dialyse est comprise entre 20 et 40 mg/g.

Dans un mode de réalisation, le polyol est le sorbitol et la concentration massique en sorbitol dans la solution de dialyse est comprise entre 25 et 35 mg/g.

Dans un mode de réalisation, le polyol est le sorbitol et la concentration massique en sorbitol dans la solution de dialyse est de 35 mg/g.

Dans un mode de réalisation, le polyol est le maltitol et la concentration massique en maltitol dans la solution de dialyse est comprise entre 10 et 40 mg/g.

Dans un mode de réalisation, le polyol est le maltitol et la concentration massique en maltitol dans la solution de dialyse est comprise entre 15 et 30 mg/g.

Dans un mode de réalisation, le polyol est le maltitol et la concentration massique en maltitol dans la solution de dialyse est comprise entre 15 et 25 mg/g.

Dans un mode de réalisation, le polyol est le maltitol et la concentration massique en maltitol dans la solution de dialyse est comprise entre 20 et 40 mg/g.

Dans un mode de réalisation, le polyol est le maltitol et la concentration massique en maltitol dans la solution de dialyse est comprise entre 25 et 35 mg/g.

Dans un mode de réalisation, le polyol est le maltitol et la concentration massique en maltitol dans la solution de dialyse est de 35 mg/g.

Dans un mode de réalisation, le polyol est le glycérol et la concentration massique en glycérol dans la solution de dialyse est comprise entre 10 et 40 mg/g.

Dans un mode de réalisation, le polyol est le glycérol et la concentration massique en glycérol dans la solution de dialyse est comprise entre 15 et 30 mg/g.

Dans un mode de réalisation, le polyol est le glycérol et la concentration massique en glycérol dans la solution de dialyse est comprise entre 15 et 25 mg/g.

Dans un mode de réalisation, le polyol est le glycérol et la concentration massique en glycérol dans la solution de dialyse est comprise entre 20 et 40 mg/g.

Dans un mode de réalisation, le polyol est le glycérol et la concentration massique en glycérol dans la solution de dialyse est comprise entre 25 et 35 mg/g.

Dans un mode de réalisation, le polyol est le glycérol et la concentration massique en glycérol dans la solution de dialyse est de 35 mg/g.

Dans un mode de réalisation, le procédé est caractérisé en ce que l'au moins un composé organique est choisi dans le groupe constitué des anesthésiques locaux.

Dans un mode de réalisation, l'anesthésique local est choisi dans le groupe des amino-esters.

Dans un mode de réalisation, l'amino-ester est choisi dans le groupe comprenant la procaïne, la benzocaïne, la chloroprocaïne et la tétracaïne sous forme de base ou de sel, par exemple sous forme de chlorhydrate.

Dans un mode de réalisation, l'anesthésique local est choisi dans le groupe des amino-amides.

Dans un mode de réalisation, l'amino-amide est choisi dans le groupe comprenant la lidocaïne, la mépivacaïne, la prilocaïne, l'articaïne, l'aptocaïne, la bupivacaïne, l'étidocaïne et la ropivacaïne sous forme de base ou de sel, par exemple sous forme de chlorhydrate.

Dans un mode de réalisation, l'anesthésique local est choisi dans le groupe des amino-éthers.

Dans un mode de réalisation, l'amino-éther est choisi dans le groupe comprenant la diamocaïne et la pramocaïne sous forme de base ou de sel, par exemple sous forme de chlorhydrate ou de cyclamate.

Dans un mode de réalisation, l'amino-éther est choisi dans le groupe constitué par la lidocaïne, la mépivacaïne, et leurs sels et leurs isomères isolés.

Dans un mode de réalisation, l'amino-éther est la lidocaïne.

Dans un mode de réalisation, l'amino-éther est la lidocaïne ou l'un de ses sels pharmaceutiquement acceptables.

Dans un mode de réalisation, l'amino-éther est le chlorhydrate de lidocaïne.

Dans un mode de réalisation, l'amino-éther est la mépivacaïne.

Dans un mode de réalisation, l'amino-éther est la mépivacaïne ou l'un de ses sels pharmaceutiquement acceptables.

Dans un mode de réalisation, l'amino-éther est choisi dans le groupe constitué par le chlorhydrate de mépivacaïne racémique, le chlorhydrate de mépivacaïne racémique, le chlorhydrate de (r)-mépivacaïne, le chlorhydrate de (s)-mépivacaïne, la (r)-mépivacaïne et la (s)-mépivacaïne, ou l'un de leurs sels pharmaceutiquement acceptables.

Dans un mode de réalisation, l'amino-éther est le chlorhydrate de mépivacaïne.

Dans un mode de réalisation, l'amino-éther est le chlorhydrate de (r)-mépivacaïne.

Dans un mode de réalisation, l'amino-éther est le chlorhydrate de (s)-mépivacaïne.

Dans un mode de réalisation, l'amino-éther est le chlorhydrate de mépivacaïne racémique.

Dans un mode de réalisation, l'amino-éther est la (r)-mépivacaïne.

Dans un mode de réalisation, l'amino-éther est la (s)-mépivacaïne.

Dans un mode de réalisation, l'amino-éther est la mépivacaïne racémique.

Dans un mode de réalisation, l'anesthésique local est la dyclonine sous forme de base ou de sel, par exemple sous forme de chlorhydrate.

Dans un mode de réalisation, l'anesthésique local est le chlorobutanol.

Dans un mode de réalisation, l'anesthésique local est le guaféçaïnol.

Dans un mode de réalisation, l'anesthésique local est le polidocanol.

Dans un mode de réalisation, la concentration massique en anesthésique local dans la solution de dialyse est comprise entre 0,01 mg/g et 10 mg/g.

Dans un mode de réalisation, la concentration massique en anesthésique local dans la solution de dialyse est comprise entre 0,01 et 7 mg/g.

Dans un mode de réalisation, la concentration massique en anesthésique local dans la solution de dialyse est comprise entre 0,01 et 5 mg/g.

Dans un mode de réalisation, la concentration massique en anesthésique local dans la solution de dialyse est comprise entre 0,01 et 2 mg/g.

Dans un mode de réalisation, la concentration massique en anesthésique local dans la solution de dialyse est comprise entre 0,01 et 1 mg/g.

Dans un mode de réalisation, la concentration massique en anesthésique local dans la solution de dialyse est comprise entre 0,01 et 0,7 mg/g.

Dans un mode de réalisation, la concentration massique en anesthésique local dans la solution de dialyse est comprise entre 0,01 et 0,5 mg/g.

Dans un mode de réalisation, la concentration massique en anesthésique local dans la solution de dialyse est 0,3 mg/g.

Dans un mode de réalisation, l'anesthésique local est la lidocaïne et la concentration massique en lidocaïne dans la solution de dialyse est comprise entre 0,01 mg/g et 10 mg/g.

Dans un mode de réalisation, l'anesthésique local est la lidocaïne et la concentration massique en lidocaïne dans la solution de dialyse est comprise entre 0,01 et 7 mg/g.

Dans un mode de réalisation, l'anesthésique local est la lidocaïne et la concentration massique en lidocaïne dans la solution de dialyse est comprise entre 0,01 et 5 mg/g.

Dans un mode de réalisation, l'anesthésique local est la lidocaïne et la concentration massique en lidocaïne dans la solution de dialyse est comprise entre 0,01 et 2 mg/g.

Dans un mode de réalisation, l'anesthésique local est la lidocaïne et la concentration massique en lidocaïne dans la solution de dialyse est comprise entre 0,01 et 1 mg/g.

Dans un mode de réalisation, l'anesthésique local est la lidocaïne et la concentration massique en lidocaïne dans la solution de dialyse est comprise entre 0,01 et 0,7 mg/g.

Dans un mode de réalisation, l'anesthésique local est la lidocaïne et la concentration massique en lidocaïne dans la solution de dialyse est comprise entre 0,01 et 0,5 mg/g.

Dans un mode de réalisation, l'anesthésique local est la lidocaïne et la concentration massique en lidocaïne dans la solution de dialyse est 0,3 mg/g.

Dans un mode de réalisation, l'anesthésique local est la mépivacaïne et la concentration massique en mépivacaïne dans la solution de dialyse est comprise entre 0,01 mg/g et 10 mg/g.

Dans un mode de réalisation, l'anesthésique local est la mépivacaïne et la concentration massique en mépivacaïne dans la solution de dialyse est comprise entre 0,01 et 7 mg/g.

Dans un mode de réalisation, l'anesthésique local est la mépivacaïne et la concentration massique en mépivacaïne dans la solution de dialyse est comprise entre 0,01 et 5 mg/g.

Dans un mode de réalisation, l'anesthésique local est la mépivacaïne et la concentration massique en mépivacaïne dans la solution de dialyse est comprise entre 0,01 et 2 mg/g.

Dans un mode de réalisation, l'anesthésique local est la mépivacaïne et la concentration massique en mépivacaïne dans la solution de dialyse est comprise entre 0,01 et 1 mg/g.

Dans un mode de réalisation, l'anesthésique local est la mépivacaïne et la concentration massique en mépivacaïne dans la solution de dialyse est comprise entre 0,01 et 0,7 mg/g.

Dans un mode de réalisation, l'anesthésique local est la mépivacaïne et la concentration massique en mépivacaïne dans la solution de dialyse est comprise entre 0,01 et 0,5 mg/g.

Dans un mode de réalisation, l'anesthésique local est la mépivacaïne et la concentration massique en mépivacaïne dans la solution de dialyse est 0,3 mg/g.

Dans un mode de réalisation, l'anesthésique local est la prilocaïne.

Dans un mode de réalisation, l'anesthésique local est la prilocaïne ou l'un de ses sels pharmaceutiquement acceptables.

Dans un mode de réalisation, l'anesthésique local est le chlorhydrate de prilocaïne.

Dans un mode de réalisation, l'anesthésique local est la prilocaïne et la concentration massique en prilocaïne dans la solution de dialyse est comprise entre 0,01 mg/g et 10 mg/g.

Dans un mode de réalisation, l'anesthésique local est la prilocaïne et la concentration massique en prilocaïne dans la solution de dialyse est comprise entre 0,01 et 7 mg/g.

Dans un mode de réalisation, l'anesthésique local est la prilocaïne et la concentration massique en prilocaïne dans la solution de dialyse est comprise entre 0,01 et 5 mg/g.

Dans un mode de réalisation, l'anesthésique local est la prilocaïne et la concentration massique en prilocaïne dans la solution de dialyse est comprise entre 0,01 et 2 mg/g.

Dans un mode de réalisation, l'anesthésique local est la prilocaïne et la concentration massique en prilocaïne dans la solution de dialyse est comprise entre 0,01 et 1 mg/g.

Dans un mode de réalisation, l'anesthésique local est la prilocaïne et la concentration massique en prilocaïne dans la solution de dialyse est comprise entre 0,01 et 0,7 mg/g.

Dans un mode de réalisation, l'anesthésique local est la prilocaïne et la concentration massique en prilocaïne dans la solution de dialyse est comprise entre 0,01 et 0,5 mg/g.

Dans un mode de réalisation, l'anesthésique local est la prilocaïne et la concentration massique en prilocaïne dans la solution de dialyse est 0,3 mg/g.

Dans un mode de réalisation, le procédé est caractérisé en ce que l'au moins un composé organique est choisi dans le groupe constitué des vitamines.

Dans un mode de réalisation, les vitamines sont choisies dans le groupe comprenant le rétinol, la thiamine, la riboflavine, la nicotinamide, le dexpenthénol, la piridoxine, l'acide ascorbique, l'ergocalciférol, le tocophérol, la biotine et l'acide folique seuls ou en mélange.

Dans un mode de réalisation, la concentration en vitamines est comprise entre 0,01 et 200 mg/g.

Dans un mode de réalisation, la concentration en vitamines est comprise entre 0,1 et 100 mg/g.

Dans un mode de réalisation, la concentration en vitamines est comprise entre 0,5 et 50 mg/g.

Dans un mode de réalisation, le procédé est caractérisé en ce que l'au moins un composé organique est choisi dans le groupe constitué des acides aminés.

Dans un mode de réalisation, le procédé est caractérisé en ce que l'au moins un composé organique est choisi dans le groupe constitué des acides aminés, acides aminés essentiels, acides aminés semi-essentiels et ou acides aminés non essentiels, seuls ou en mélange.

Dans un mode de réalisation, les acides aminés sont choisis dans le groupe des acides aminés essentiels.

Dans un mode de réalisation, les acides aminés essentiels sont choisis dans le groupe consititué de l'isoleucine, la leucine, la lysine, la méthionine, la phénylalanine, la thréonine, le tryptophane et la valine, seuls ou en mélange.

Dans un mode de réalisation, les acides aminés sont chosis dans le groupe des acides aminés semi-essentiels.

Dans un mode de réalisation, les acides aminés semi-essentiels sont choisis dans le groupe consititué de l'arginine et de l'histidine, seuls ou en mélange.

Dans un mode de réalisation, les acides aminés sont choisis dans le groupe des acides aminés non essentiels.

Dans un mode de réalisation, les acides aminés non essentiels sont choisis dans le groupe consititué par l'alanine, l'asparagine, l'acide aspartique, la cystéine, la glutamine,l'acide glutamique, la glycine,la proline, la sérine, la tyrosine, seuls ou en mélange.

Dans un mode de réalisation les acides aminés sont choisis dans le groupe comprenant l'hydroxyproline, la taurine et l'ornithine seuls ou en mélange.

Dans un mode de réalisation, la concentration en acides aminés est comprise entre 0,01 et 150 mg/g.

Dans un mode de réalisation, la concentration en acides aminés est comprise entre 0,1 et 100 mg/g.

Dans un mode de réalisation, la concentration en acides aminés est comprise entre 0,5 et 50 mg/g.

Dans un mode de réalisation, le procédé est caractérisé en ce que l'au moins un composé organique est choisi dans le groupe constitué des vasoconstricteurs.

Dans un mode de réalisation, les vasoconstricteurs sont choisis dans le groupe comprenant la naphazoline, l'épinéphrine, la méthoxamine, la méthylnorépinéphrine, la norépinéphrine, l'oxyméthazoline, la phényléphrine, la pseudoéphédrine, la synéphrine, la cirazoline ou la xylométhazoline.

Dans un mode de réalisation, la concentration en vasoconstricteurs est comprise entre 0,01 et 3 mg/g.

Dans un mode de réalisation, le procédé est caractérisé en ce que l'au moins un composé organique est choisi dans le groupe constitué des vasodilatateurs.

Dans un mode de réalisation, les vasodilatateurs sont choisis dans le groupe comprenant l'adénosine, l'acide nicotinique, le minoxidil et le diazoxide seuls ou en mélange.

Dans un mode de réalisation, la concentration en vasodilatateurs est comprise entre 0,01 et 10 mg/g.

Dans un mode de réalisation, le procédé est caractérisé en ce que l'au moins un composé organique est choisi dans le groupe constitué des antihémorragiques ou hémostatiques.

Dans un mode de réalisation, les anti-hémorragiques ou hémostatiques sont choisis dans le groupe comprenant l'acide aminocaproïque ou l'acide tranexamique, seuls ou en mélange.

Dans un mode de réalisation, la concentration en anti-hémorragiques ou hémostatiques est comprise entre 0,01 et 5 mg/g.

Dans un mode de réalisation, le procédé est caractérisé en ce que l'au moins un composé organique est choisi dans le groupe constitué des anti-inflammatoires non-stéroïdiens.

Dans un mode de réalisation, les anti-inflammatoires sont choisis dans le groupe comprenant les antiinflammatoires salicylés, les dérivés propioniques, les dérivés indoliques, les dérivés pyrazolés, les oxicams, les coxibs.

Dans un mode de réalisation, les anti-inflammatoires sont choisis dans le groupe comprenant le diclofénac, le nimésulfide, l'acide niflumique, l'acide méfénamique et la nabumétone seuls ou en mélange.

Dans un mode de réalisation, les anti-inflammatoires sont choisis dans le groupe des antiinflammatoires salicylés comprenant le diflunisal, le bénorilate et l'aspirine seuls ou en mélange.

Dans un mode de réalisation, les anti-inflammatoires sont choisis dans le groupe des dérivés propioniques comprenant l'alminoprofène, le kétoprofène, l'ibuprofène, le naproxène, le flurbiprofène et l'acide tiaprofènique seuls ou en mélange.

Dans un mode de réalisation, les anti-inflammatoires sont choisis dans le groupe des dérivés indoliques comprenant l'indométacine, le sulindac et l'étodolac seuls ou en mélange.

Dans un mode de réalisation, les anti-inflammatoires sont choisis dans le groupe des dérivés pyrazolés comprenant notamment la phénylbutazone.

Dans un mode de réalisation, les anti-inflammatoires sont choisis dans le groupe des oxicams comprenant le piroxicam, le ténoxicam et le méloxicam seuls ou en mélange.

Dans un mode de réalisation, les anti-inflammatoires sont choisis dans le groupe des coxibs comprenant le célécoxib, l'étoricoxib et le rofécoxib seuls ou en mélange.

Dans un mode de réalisation, la concentration en anti-inflammatoires non-stéroïdiens est comprise entre 0,01 et 2000 mg/g.

Dans un mode de réalisation, la concentration en anti-inflammatoires non-stéroïdiens est comprise entre 0,1 et 1000 mg/g.

Dans un mode de réalisation, la concentration en anti-inflammatoires non-stéroïdiens est comprise entre 0,5 et 500 mg/g.

Dans un mode de réalisation, le procédé est caractérisé en ce que l'au moins un composé organique est choisi dans le groupe constitué des antimicrobiens.

Dans un mode de réalisation, les antimicrobiens sont choisis dans le groupe comprenant la gentamicine, la sulfadiazine d'argent, le métronidazole, la fucidine, la bacitracine, l'éosine, la povidone iodée, le gluconate de cuivre, le gluconate de zinc, le gluconate de manganèse ou leur sels, seuls ou en mélange.

Dans un mode de réalisation, la concentration en antimicrobiens est comprise entre 0,1 et 200 mg/g.

Dans un mode de réalisation, la concentration en antimicrobiens est comprise entre 0,5 et 100 mg/g.

Dans un mode de réalisation, le procédé est caractérisé en ce que l'au moins un composé organique est choisi dans le groupe constitué des glycosides et leurs dérivés.

Dans un mode de réalisation, les glycosides sont choisis dans le groupe comprenant le D-glucopyranose, le 1,4 glycoside, l'esculine, l'hespéridine, la diosmine, l'arbutine, la skimmine ou l'aloïne, seuls ou en mélanges.

Dans un mode de réalisation, la concentration en glycosides est comprise entre 0,1 et 200 mg/g.

Dans un mode de réalisation, la concentration en glycosides est comprise entre 0,5 et 100 mg/g.

Dans un mode de réalisation, le procédé est caractérisé en ce que l'au moins un composé organique est choisi dans le groupe constitué des hydratants ou régénérants tissulaires.

Dans un mode de réalisation, les hydratants ou régénérants tissulaires sont choisis dans le groupe constitué des macro-éléments.

Dans un mode de réalisation les macro-éléments sont choisis dans le groupe comprenant les gluconates de fer, calcium, cuivre, zinc, manganèse, magnésium ou potassium, du timéthylsilanol, du triméthylsilanolate, du triméthylsilanolate de potassium, du méthylsilanol mannuronate, du monoéthyltrisilanol orthohydroxybenzoate de sodium sels ou en mélanges.

Dans un mode de réalisation, les hydratants ou régénérants tissulaires sont choisis dans le groupe constitué des acides aminés essentiels.

Dans un mode de réalisation, les acides aminés essentiels sont choisis dans le groupe constitué de l'isoleucine, la leucine, la lysine, la méthionine, la phénylalanine, la thréonine, le tryptophane et la valine, seuls ou en mélange.

Dans un mode de réalisation, les hydratants ou régénérants tissulaires sont choisis dans le groupe constitué des acides aminés semi-essentiels.

Dans un mode de réalisation, les acides aminés semi-essentiels sont choisis dans le groupe constitué de l'arginine et de l'histidine, seuls ou en mélange.

Dans un mode de réalisation, les hydratants ou régénérants tissulaires sont choisis dans le groupe constitué des acides aminés non essentiels.

Dans un mode de réalisation, les acides aminés non essentiels sont choisis dans le groupe constitué par l'alanine, l'asparagine, l'acide aspartique, la cystéine, la glutamine, l'acide glutamique, la glycine, la proline, la sérine, la tyrosine, seuls ou en mélange.

Dans un mode de réalisation les acides aminés sont choisis dans le groupe comprenant l'hydroxyproline, la taurine et l'ornithine seuls ou en mélange.

Dans un mode de réalisation, les hydratants ou régénérants tissulaires sont choisis dans le groupe constitué des vitamines.

Dans un mode de réalisation, les vitamines sont choisies dans le groupe constitué du rétinol, de la thiamine, la riboflavine, du nicotinamide, de l'adénine, du pantothénate de calcium, de la pyridoxine, de l'inositol, de la biotine, de l'acide folique, de l'acides para-amino-benzoïque, de la cobalamine, de la vitamine C, du chlorure de choline, seuls ou en mélanges.

Dans un mode de réalisation, les hydratants ou régénérants tissulaires sont choisis dans le groupe constitué des acides nucléiques.

Dans un mode de réalisation, les acides nucléiques sont choisis dans le groupe constitué de la déoxyadénosine, de la déoxycytidine, de la déoxyguanosine, de la déoxythymidine, de la méthylcytosine seuls ou en mélanges.

Dans un mode de réalisation, les hydratants ou régénérants tissulaires sont choisis dans le groupe constitué des coenzymes.

Dans un mode de réalisation, les coenzymes sont choisis dans le groupe constitué de la thiamine pyrophosphate, du coenzyme A, du FAD, du NAD, DU NADP, de l'UTP seules ou en mélanges.

Dans un mode de réalisation, les hydratants ou régénérants tissulaires sont choisis dans le groupe constitué de la déoxythymidine, du glutathion, du pyruvate de sodium, de l'acide lipoïque et de la putrescine, seuls ou en mélange.

Dans un mode de réalisation, les régénérants tissulaires sont choisis dans le groupe constitué des macro-éléments.

Dans un mode de réalisation les macro-éléments sont choisis dans le groupe comprenant les gluconates de fer, calcium, cuivre, zinc, manganèse, magnésium ou potassium, du timéthylsilanol, du triméthylsilanolate, du triméthylsilanolate de potassium, du méthylsilanol mannuronate, du monoéthyltrisilanol orthohydroxybenzoate de sodium sels ou en mélanges

Dans un mode de réalisation, la concentration en hydratants ou régénérants tissulaires est comprise entre 0,01 et 500 mg/g.

Dans un mode de réalisation, la concentration en hydratants ou régénérants tissulaires est comprise entre 0,1 et 200 mg/g.

Dans un mode de réalisation, le procédé est caractérisé en ce qu'au moins un composé organique est choisi dans le groupe des antioxydants et un composé organique est choisi dans le groupe des anesthésiques locaux.

Dans un mode de réalisation l'antioxydant est choisi dans le groupe des polyols et l'anesthésique local est choisi dans le groupe des amino-amides.

Dans un mode de réalisation, le procédé est caractérisé en ce qu'au moins un composé organique est choisi dans le groupe des antioxydants, au moins un composé organique est choisi dans le groupe des vitamines et au moins un composé est choisi dans le groupe des tissulaires.

Dans un mode de réalisation, le procédé est caractérisé en ce qu'au moins un composé organique est choisi dans le groupe des acides aminés, au moins un composé organique est choisi dans le groupe des vitamines et au moins un composé est choisi dans le groupe des régénérants tissulaires.

Dans un mode de réalisation, le procédé est caractérisé en ce qu'au moins un composé organique est choisi dans le groupe des antioxydants, au moins un composé organique est choisi dans le groupe des acides aminés, au moins un composé organique est choisi dans le groupe des vitamines et au moins un composé est choisi dans le groupe des régénérants tissulaires.

Ledit hydrogel est constitué d'au moins un polysaccharide choisi dans le groupe constitué de l'acide hyaluronique, du kératane, de l'héparine, de la cellulose, des dérivés de cellulose (notamment l'hydroxypropylcellulose, l'hydroxypropylméthylcellulose, l'éthylméthyle cellulose, la carboxyméthylcellulose) de l'acide alginique, du xanthane, du carraghénane, du chitosan, de la chondroitine, l'héparosan, et leurs sels biologiquement acceptables, seul(s) ou en mélange.

Dans un mode de réalisation, ledit polysaccharide est de l'acide hyaluronique.

Dans un mode de réalisation, ledit polysaccharide est de l'acide hyaluronique ou l'un de ses sels, seul ou en mélange.

Dans un mode de réalisation, ledit polysaccharide est de l'acide hyaluronique sous forme de sel de sodium ou de potassium.

Dans un mode de réalisation, ledit polysaccharide est de l'acide hyaluronique sous forme de sel de sodium.

Dans un mode de réalisation, ledit polysaccharide est de l'acide hyaluronique réticulé ou l'un de ses sels, seul ou en mélange.

Dans un mode de réalisation, ledit polysaccharide est de l'acide hyaluronique réticulé ou l'un de ses sels, seul ou en mélange, et en ce que ladite réticulation est réalisée au moyen d'au moins un agent de réticulation.

Dans un mode de réalisation, ledit polysaccharide est de l'acide hyaluronique réticulé ou l'un de ses sels, seul ou en mélange, et en ce que ladite réticulation est réalisée au moyen d'au moins un agent de réticulation bi- ou polyfonctionnel.

Dans un mode de réalisation, ledit polysaccharide est de l'acide hyaluronique réticulé ou l'un de ses sels, seul ou en mélange, et en ce que ladite réticulation est réalisée au moyen d'au moins un agent de réticulation bi- ou polyfonctionnel choisi dans le groupe constitué par l'éther d'éthylèneglycoldiglycidyle, l'éther de butanedioldiglycidyle (BDDE), l'éther de polyglycérolpolyglycidyle, l'éther de polyéthylèneglycoldiglycidyle, l'éther de polypropylèneglycoldiglycidyle, un bis- ou polyépoxy tel que 1,2,3,4-diépoxybutane ou 1,2,7,8-diépoxyoctane, une dialkylsulfone, la divinylsulfone, le formaldéhyde, l'épichlorohydrine ou bien encore le glutaraldéhyde, les carbodiimides tels que par exemple le 1-éthyl-3-[3-dimethylaminopropyl]carbodiimide hydrochloride (EDC) ou les réticulants à base de dérivés de phosphore (V).

Dans un mode de réalisation, les dérivés de phosphore (V) sont choisis dans le groupe comprenant les halogènures de phosphore, l'oxyhalogènure de phosphore, l'halogène étant choisi dans le groupe comprenant le chlore, le brome ou l'iode, le pentoxyde de phosphore et les trimétaphosphates alcalins, le métal alcalin étant choisi dans le groupe comprenant le sodium et le potassium.

Dans un mode de réalisation, ledit polysaccharide est de l'acide hyaluronique réticulé ou l'un de ses sels, seul ou en mélange, et en ce que ladite réticulation est réalisée au moyen d'au moins un agent de réticulation bifonctionnel étant l'éther de butanedioldiglycidyle (BDDE) ou le 1,2,7,8-diépoxyoctane.

Dans un mode de réalisation, ledit polysaccharide est de l'acide hyaluronique réticulé ou l'un de ses sels, seul ou en mélange, et en ce que ladite réticulation est réalisée au moyen du BDDE.

Dans un mode de réalisation, ledit polysaccharide est de l'acide hyaluronique réticulé ou l'un de ses sels, seul ou en mélange, et en ce que ladite réticulation est réalisée au moyen d'un trimétaphosphate de sodium ou de potassium.

Lorsque le polysaccharide et plus particulièrement l'acide hyaluronique est réticulé au moyen d'un agent réticulant, le taux de réticulation (x) est calculé de manière théorique au moyen de la formule suivante :

$$X = \frac{\text{nombre de moles d'agent réticulant introduites dans le milieu réactionnel}}{\text{nombre de moles d'unités de répétition introduites dans le milieu réactionnel}}$$

S'agissant de l'acide hyaluronique, l'unité de répétition est un motif disaccharidique.

Dans un mode de réalisation, l'acide hyaluronique réticulé présente un taux de réticulation x compris entre 0,001 et 0,5.

Dans un mode de réalisation, l'acide hyaluronique réticulé présente un taux de réticulation x compris entre 0,01 et 0,4.

Dans un mode de réalisation, l'acide hyaluronique réticulé présente un taux de réticulation x compris entre 0,1 et 0,3.

Dans un mode de réalisation, l'acide hyaluronique réticulé présente un taux de réticulation x de 0,06.

Dans un mode de réalisation, l'acide hyaluronique réticulé présente un taux de réticulation x de 0,07.

Dans un mode de réalisation, l'acide hyaluronique réticulé présente un taux de réticulation x de 0,12,

Dans un mode de réalisation, ledit polysaccharide est de l'acide hyaluronique co-réticulé ou l'un de ses sels, seul ou en mélange.

Dans un mode de réalisation, ledit polysaccharide est de l'acide hyaluronique modifié chimiquement par substitution, réticulé ou non réticulé, ou l'un de ses sels, seul ou en mélange.

Dans un mode de réalisation, ledit polysaccharide est de l'acide hyaluronique doublement réticulé tel que décrit dans la demande de brevet WO2000/046253 au nom de Fermentech medical limited.

Dans un mode de réalisation, ledit polysaccharide est un mélange d'acides hyaluroniques, ou l'un de leurs sels, réticulé et non réticulé.

Dans un mode de réalisation, ledit polysaccharide est un mélange d'acides hyaluroniques, ou l'un de leurs sels, réticulés.

Dans un mode de réalisation, ledit polysaccharide est un mélange d'acides hyaluroniques, ou l'un de leurs sels, réticulés tel que celui décrit dans la demande de brevet WO2009/071697 au nom de la demanderesse.

Dans un mode de réalisation, ledit polysaccharide est un mélange d'acides hyaluroniques, obtenu par mélange de plusieurs acides hyaluroniques, ou l'un de leurs sels, de masses moléculaires différentes préalablement à leur réticulation, tel que décrit dans la demande de brevet WO2004092222 au nom de Cornéal industrie.

Dans un mode de réalisation, ledit polysaccharide est de l'acide hyaluronique ou l'un de ses sels, substitué par un groupement apportant des propriétés lipophile ou hydratante, comme par exemple les acides hyaluroniques substitués tels que décrits dans la demande de brevet FR2983483 au nom de la demanderesse. Dans cette demande est décrit un procédé de substitution et réticulation simultanées d'un polysaccharide via ses fonctions hydroxyles, en phase aqueuse, caractérisé en ce qu'il comprend les étapes suivantes : (i) on dispose d'un polysaccharide en milieu aqueux, (ii) on le met en présence d'au moins un précurseur d'un substituant, (iii) on le met en presence d'un agent de réticulation, et (iv) on obtient et on isole le polysaccharide substitué et réticulé. Ces étapes de procédé peuvent parfaitement être mises en oeuvre dans le cadre de la présente invention.

Dans un mode de réalisation, ledit polysaccharide est de l'acide hyaluronique ou l'un de ses sels, greffé avec du glycérol, par exemple tel que décrit dans la demande WO2017162676 au nom de MERZ.

Dans un mode de réalisation, la masse moléculaire mw de l'au moins un acide hyaluronique est comprise dans un intervalle de 0,01 MDa et 5 MDa.

Dans un mode de réalisation, la masse moléculaire mw de l'au moins un acide hyaluronique est comprise dans un intervalle de 0,1 MDa et 3,5 MDa.

Dans un mode de réalisation, la masse moléculaire mw de l'au moins un acide hyaluronique est comprise dans un intervalle de 1 MDa et 3 MDa.

Dans un mode de réalisation, la masse moléculaire mw de l'au moins un acide hyaluronique est de 1 MDa.

Dans un mode de réalisation, la masse moléculaire mw de l'au moins un acide hyaluronique est de 3 MDa.

Dans un mode de réalisation, la concentration en acide hyaluronique [HA] est comprise entre 2 mg/g et 50 mg/g de poids total dudit l'hydrogel.

Dans un mode de réalisation, la concentration en acide hyaluronique [HA] est comprise entre 4 mg/g et 40 mg/g de poids total dudit l'hydrogel.

Dans un mode de réalisation, la concentration en acide hyaluronique [HA] est comprise entre 5 mg/g et 30 mg/g de poids total dudit l'hydrogel.

Dans un mode de réalisation, la concentration en acide hyaluronique [HA] est comprise entre 10 mg/g et 30 mg/g de poids total dudit l'hydrogel.

Dans un mode de réalisation, la concentration en acide hyaluronique [HA] est de 20 mg/g de poids total dudit l'hydrogel.

Dans un mode de réalisation, l'hydrogel est stérilisé, c'est-à-dire qu'il subit après sa préparation une étape de stérilisation, ladite étape de stérilisation étant effectuée par la chaleur, par la chaleur humide, par le rayonnement gamma($\gamma$), par faisceau d'électrons accélérés (electron-beam), ou encore par filtration stérilisante (0,22 $\mu$m) s'agissant des hydrogels à base d'acide hyaluronique non réticulé.

Dans un mode de réalisation, ladite étape de stérilisation est effectuée par autoclavage à la vapeur.

Dans un mode de réalisation, l'autoclavage à la vapeur est réalisé à une température de 121 à 134°c, pendant une durée adaptée à la température.

Dans un mode de réalisation, l'autoclavage à la vapeur est réalisé à une température comprise entre 127 et 130°c pendant une durée comprise entre 1 et 20 min.

Dans un mode de réalisation, la valeur stérilisatrice F0, est supérieure à 1 min à 121°C.

Dans un mode de réalisation, ladite étape de stérilisation est effectuée par irradiation par rayonnements gamma ($\gamma$).

Les applications visées sont plus particulièrement les applications communément répandues dans le cadre des viscoélastiques injectables et des polysaccharides utilisés ou potentiellement utilisables dans les pathologies ou traitements suivants :

- Injections esthétiques au niveau du visage : de mésothérapie, de comblement de rides, défauts cutanés ou volumatrices (pommettes, menton, lèvres) ;
- Injections volumatrices au niveau du corps : augmentation des seins et des fesses, augmentation du point G, vaginoplastie, reconstruction des lèvres vaginales, augmentation de la taille du pénis ;
- Traitement de l'arthrose, injection dans l'articulation en remplacement ou en complément du liquide synovial déficient ;
- Injection péri-urétrale pour le traitement de l'incontinence urinaire par insuffisance sphinctérienne ;
- Injection post-chirurgicale pour éviter les adhérences, péritonéales notamment ;
- Injection suite à une chirurgie de la presbytie par incisions sclérales au laser ;
- Injection dans la cavité vitréenne ;
- Injection au cours de la chirurgie de la cataracte ;
- Injection dans les parties génitales ;
- Injection pour l'espacement tissulaire.

Plus particulièrement, en chirurgie esthétique, en fonction de ses propriétés viscoélastiques et de rémanence, la composition aqueuse stérilisée obtenue selon le procédé de l'invention pourra être utilisée :

- pour améliorer l'hydratation et la qualité de la peau par la mésothérapie cutanée du visage, cou, décolleté ou des mains. Dans ce cas le produit est injecté à l'aide d'aiguille de diamètre variant entre 27 et 32G et des longueurs variant généralement entre 1/8" et ½". La composition pourra être injectée soit à la main, soit par l'intermédiaire de dispositifs automatiques comme les stylos injecteurs.
- pour le comblement des rides fines, moyennes ou profondes, et être injectée avec des aiguilles de diamètre fin (27 Gauge à 30G le plus majoritairement, les longueurs étant généralement de 1/2" );
- comme volumateur avec une injection soit par des aiguilles de diamètre plus important, de 25 à 27G Gauge par exemple, et plus longues (30 à 40 mm par exemple), soit par des canules de longueur majoritairement comprise entre 40 et 70mm et de diamètre 22 à 25G; dans ce cas, son caractère cohésif permettra de garantir son maintien à l'emplacement de l'injection.

La composition aqueuse stérilisée selon l'invention trouve également une application importante en chirurgie articulaire et en chirurgie dentaire pour le comblement des poches parodontales par exemple.

Ces exemples d'utilisation ne sont nullement limitatifs, la composition aqueuse stérilisée selon la présente invention étant plus largement prévue pour :

- combler des volumes ;
- générer des espaces entre certains tissus ;
- remplacer des liquides physiologiques déficients.

L'invention concerne également un kit comprenant une composition aqueuse stérilisée selon l'invention, conditionnée en seringues et stérilisées après conditionnement.

L'invention concerne également un kit comprenant une composition aqueuse stérilisée selon l'invention, conditionnée en flacons uni-doses et stérilisée après conditionnement.

Exemples

Fabrication des gels

- Gels d'acide hyaluronique réticulé

Les gels comprenant de l'acide hyaluronique réticulé sont obtenus selon le mode opératoire décrit dans la demande de brevet WO 2009/071697 au nom de la demanderesse à partir de fibres de hyaluronate de sodium (NaHA) et d'éther de butanedioldiglycidyle (BDDE).

- Gels d'acide hyaluronique réticulé et interpénétré

Les gels comprenant de l'acide hyaluronique réticulé et interpénétrés sont obtenus selon le mode opératoire décrit dans la demande de brevet WO 2009/071697 au nom de la demanderesse.

- Gels d'acide hyaluronique co-réticulé

Les gels comprenant de l'acide hyaluronique co-réticulé sont obtenus selon le mode opératoire décrit dans la demande de brevet WO 86/00079 au nom de ALLERGAN.

- Stérilisation

Les compositions ainsi obtenues sont conditionnées en seringues qui sont stérilisées par autoclavage à la vapeur (T=121°C, 10 min).

Exemple 1

Incorporation de lidocaïne dans un gel d'acide hyaluronique par la dialyse.

Des fibres de hyaluronate de sodium (HA) de masse moléculaire d'environ 3 OOOkDa et contenant 12% d'humidité ont été hydratées dans la soude à 0,25N jusqu'à dissolution complète.

Puis le BDDE est introduit dans le milieu réactionnel.

Le milieu réactionnel est homogénéisé en alternant agitation manuelle à la spatule et repos pendant environ 50 minutes.

Le milieu réactionnel composé de 3g de hyaluronate de sodium, 0,23g de BDDE et 24,5g de soude à 0,25N est ensuite introduit pendant 3 heures dans un bain-marie préalablement thermostaté à 50°C.

A l'issue de la réticulation, le milieu réactionnel est neutralisé par 5,5g de HCl 1N et 30g de tampon phosphate (PBS). Après homogénéisation à la spatule, le pH est mesuré à 7,2.

Une solution saline de chlorhydrate de lidocaïne a été préparée en dissolvant les composés suivants :

$NaH_2PO_4, 2H_2O$            0,0459g

(suite)

| Na$_2$HPO$_4$ | 0,2690g |
| NaCl | 7,99g |
| Chlorhydrate de lidocaïne | 3,01g |
| EPPI | qsp 1L |

Cette solution titre 0,28% en lidocaïne et présente un pH de 6,8 et une osmolarité de 274mOsm.

| Solution saline de chlrohydrate de lidocaïne | pH | 6,8 |
| --- | --- | --- |
| | Osmolarité (mOsm) | 274 |
| | Titre en lidocaïne | 0,28% |

10g de gel de HA réticulé sont chargés en membrane de dialyse tubulaire à seuil de coupure 12-14kD et dialysés à +4°C contre 90g de la solution saline de chlorhydrate de lidocaïne. 6 bains de successifs de solution saline sont réalisés pour une durée totale de 74H29.

La concentration finale en HA est de 21mg/g, le pH est de 6,7 et l'osmolarité de 292mOsm. La concentration en lidocaïne dans le gel après dialyse a été déterminée par dosage UV selon une méthode interne validée. Le titre en lidocaïne est de 0,3%.

| Gel de HA réticulé après dialyse | [HA] (mg/g) | 21,2 |
| --- | --- | --- |
| | pH | 6,7 |
| | Osmolarité (mOsm) | 292 |
| | Titre en lidocaïne | 0,31% |

Exemple 2

Incorporation de mannitol dans un gel d'acide hyaluronique par la dialyse

Des fibres de hyaluronate de sodium (HA) de masse moléculaire d'environ 3 OOOkDa et contenant 10,3% d'humidité ont été hydratées dans la soude à 0,25N jusqu'à dissolution complète.

Puis le BDDE sont introduit dans le milieu réactionnel.

Le milieu réactionnel est homogénéisé en alternant agitation mécanique et repos pendant environ 50 minutes.

Le milieu réactionnel composé de 251g de hyaluronate de sodium, 19g de BDDE et 1751g de soude à 0,25N est ensuite introduit pendant 3 heures dans un bain-marie préalablement thermostaté à 50°C.

A l'issue de la réticulation, le milieu réactionnel est neutralisé par 405g de HCl 1N et 2702g de tampon phosphate (PBS) et homogénéisé mécaniquement. Le gel obtenu est identifié M1.

En parallèle, des fibres de hyaluronate de sodium (HA) de masse moléculaire d'environ 1 OOOkDa et contenant 8% d'humidité ont été hydratées dans la soude à 0,25N jusqu'à dissolution complète.

Puis le BDDE sont introduit dans le milieu réactionnel.

Le milieu réactionnel est homogénéisé en alternant agitation mécanique et repos pendant environ 50 minutes.

Le milieu réactionnel composé de 245g de hyaluronate de sodium, 12g de BDDE et 1309g de soude à 0,25N est ensuite introduit pendant 2 heures 45 minutes dans un bain-marie préalablement thermostaté à 50°C.

A l'issue de la réticulation, le milieu réactionnel est neutralisé par 300g de HCl 1N et 2701g de tampon phosphate (PBS)et homogénéisé mécaniquement. Le gel obtenu est identifié M2.

Une solution saline de mannitol a été préparée en dissolvant les composés suivants :

| NaH$_2$PO$_4$, 2H$_2$O | 5,4g |
| Na$_2$HPO$_4$ | 67,6g |
| NaCl | 240g |
| Mannitol | 4200g |
| EPPI | qsp 120L |

Cette solution titre 35,0g/L en mannitol.

5468g de M1 sont chargés en membrane de dialyse tubulaire à seuil de coupure 12-14kD et dialysés à +10°C contre la solution saline de mannitol dans un ratio 1/11. 4 bains de successifs de solution saline de mannitol sont réalisés pour une durée totale de 40H33.

En parallèle, 5426g de M2 sont chargés en membrane de dialyse tubulaire à seuil de coupure 12-14kD et dialysés à +10°C contre la solution saline de mannitol dans un ratio 1/11. 4 bains de successifs de solution saline de mannitol sont réalisés pour une durée totale de 40H33.

A l'issue des 2 dialyses, 3234g de M1 dialysé et 3234g de M2 dialysé sont mélangés à 2134g de solution saline de mannitol. Après homogénéisation mécanique et débullage, le gel obtenu est réparti en seringues de 1mL puis stérilisées à la vapeur à 127°C avec une F0 de 21 minutes.

Le mannitol est dosé par HPLC-RID suivant une méthode interne validée. Le titre en mannitol est de 30,5 g/L.

En conclusion, la concentration en composé actif dans chaque gel correspond à la concentration dudit actif au sein du liquide de dialyse utilisé.

## Revendications

1. Procédé d'incorporation d'au moins un composé organique au sein d'un hydrogel, **caractérisé en ce que** ladite incorporation est effectuée par dialyse, ledit composé organique étant solubilisé dans la solution de dialyse à une concentration égale ou supérieure à la concentration cible en composé organique dans le gel après incorporation, ladite concentration cible étant comprise entre 0,01 et 500 mg/g.

2. Procédé selon la revendication 1, **caractérisé en ce que** la solution de dialyse est de l'eau ou une solution aqueuse qui peut comprendre en outre des sels minéraux.

3. Procédé selon l'une quelconque des revendications précédentes **caractérisé en ce que** la solution de dialyse comprend en outre du chlorure de sodium.

4. Procédé selon l'une quelconque des revendications précédentes, **caractérisé en ce que** la solution de dialyse comprend en outre un tampon pH.

5. Procédé selon l'une quelconque des revendications précédentes **caractérisé en ce que** ladite dialyse est mise en oeuvre en utilisant une membrane dont le seuil de coupure (MWCO) est compris entre 1 et 200kD (1≤ MCWO ≤ 200 kD).

6. Procédé selon l'une quelconque des revendications précédentes, **caractérisé en ce que** ladite dialyse est mise en oeuvre en milieu clos.

7. Procédé selon l'une quelconque des revendications 1 à 5, **caractérisé en ce que** ladite dialyse est mise en oeuvre en continu par mise en oeuvre d'un flux continu de solution de dialyse à travers l'hydrogel et la membrane.

8. Procédé selon l'une quelconque des revendications précédentes, **caractérisé en ce que** l'au moins un composé organique est choisi dans le groupe constitué des antioxydants.

9. Procédé selon l'une quelconque des revendications 1 à 7, **caractérisé en ce que** l'au moins un composé organique est choisi dans le groupe constitué des anesthésiques locaux.

10. Procédé selon l'une quelconque des revendications 1 à 7, **caractérisé en ce que** l'au moins un composé organique est choisi dans le groupe constitué des vitamines.

11. Procédé selon l'une quelconque des revendications 1 à 7, **caractérisé en ce que** l'au moins un composé organique est choisi dans le groupe constitué des acides aminés.

12. Procédé selon l'une quelconque des revendications 1 à 7, **caractérisé en ce que** l'au moins un composé organique est choisi dans le groupe constitué des vasoconstricteurs.

13. Procédé selon l'une quelconque des revendications 1 à 7, **caractérisé en ce que** l'au moins un composé organique est choisi dans le groupe constitué des vasodilatateurs.

**14.** Procédé selon l'une quelconque des revendications 1 à 7, **caractérisé en ce que** l'au moins un composé organique est choisi dans le groupe constitué des anti-hémorragiques ou hémostatiques.

**15.** Procédé selon l'une quelconque des revendications 1 à 7, **caractérisé en ce que** l'au moins un composé organique est choisi dans le groupe constitué des anti-inflammatoires non stéroïdiens.

**16.** Procédé selon l'une quelconque des revendications 1 à 7, **caractérisé en ce que** l'au moins un composé organique est choisi dans le groupe constitué des antimicrobiens.

**17.** Procédé selon l'une quelconque des revendications 1 à 7, **caractérisé en ce que** l'au moins un composé organique est choisi dans le groupe constitué des glycosides et leurs dérivés.

**18.** Procédé selon l'une quelconque des revendications 1 à 7, **caractérisé en ce que** l'au moins un composé organique est choisi dans le groupe constitué des hydratants ou régénérants tissulaires.

**19.** Procédé selon l'une quelconque des revendications précédentes, **caractérisé en ce que** l'hydrogel est constitué d'au moins un polysaccharide choisi dans le groupe constitué de l'acide hyaluronique, du kératane, de l'héparine, de la cellulose, des dérivés de cellulose, de l'acide alginique, du xanthane, du carraghénane, du chitosan, de la chondroitine, l'héparosan, et leurs sels biologiquement acceptables, seul(s) ou en mélange.

**20.** Procédé selon la revendication 19, **caractérisé en ce que** ledit polysaccharide est de l'acide hyaluronique.


**Patentansprüche**

**1.** Verfahren zum Einbauen von wenigstens einer organischen Verbindung in ein Hydrogel, **dadurch gekennzeichnet, dass** das Einbauen mittels Dialyse erfolgt, wobei die organische Verbindung in der Dialyselösung in einer Konzentration gelöst ist, die gleich der oder größer ist die Zielkonzentration der organischen Verbindung in dem Gel nach dem Einbauen, wobei die Zielkonzentration zwischen 0,01 und 500 mg/g beträgt.

**2.** Verfahren nach Anspruch 1, **dadurch gekennzeichnet, dass** die Dialyselösung Wasser oder eine wässrige Lösung ist, die zusätzlich Mineralsalze umfassen kann.

**3.** Verfahren nach einem der vorangehenden Ansprüche, **dadurch gekennzeichnet, dass** die Dialyselösung zusätzlich Natrimchlorid umfasst.

**4.** Verfahren nach einem der vorangehenden Ansprüche, **dadurch gekennzeichnet, dass** die Dialyselösung zusätzlich einen pH-Puffer umfasst.

**5.** Verfahren nach einem der vorangehenden Ansprüche, **dadurch gekennzeichnet, dass** die Dialyse durchgeführt wird unter Verwendung einer Membran, deren Ausschlussgröße (MWCO) zwischen 1 und 200 kD ($1 \leq MCWO \leq 200$ kD) beträgt.

**6.** Verfahren nach einem der vorangehenden Ansprüche, **dadurch gekennzeichnet, dass** die Dialyse in einem geschlossenen System durchgeführt wird.

**7.** Verfahren nach einem der Ansprüche 1 bis 5, **dadurch gekennzeichnet, dass** die Dialyse kontinuierlich durchgeführt wird, indem ein kontinuierlicher Dialyseflüssigkeitsstrom durch das Hydrogel und die Membran geführt wird.

**8.** Verfahren nach einem der vorangehenden Ansprüche, **dadurch gekennzeichnet, dass** die wenigstens eine organische Verbindung ausgewählt ist aus der Gruppe bestehend aus Antioxidanzien.

**9.** Verfahren nach einem der Ansprüche 1 bis 7, **dadurch gekennzeichnet, dass** die mindestens eine organische Verbindung ausgewählt ist aus der Gruppe bestehend aus Lokalanästhetika.

**10.** Verfahren nach einem der Ansprüche 1 bis 7, **dadurch gekennzeichnet, dass** die wenigstens eine organische Verbindung ausgewählt ist aus der Gruppe bestehend aus Vitaminen.

**11.** Verfahren nach einem der Ansprüche 1 bis 7, **dadurch gekennzeichnet, dass** die wenigstens eine organische Verbindung ausgewählt ist aus der Gruppe bestehend aus Aminosäuren.

**12.** Verfahren nach einem der Ansprüche 1 bis 7, **dadurch gekennzeichnet, dass** die wenigstens eine organische Verbindung ausgewählt ist aus der Gruppe bestehend aus Vasokonstriktoren.

**13.** Verfahren nach einem der Ansprüche 1 bis 7, **dadurch gekennzeichnet, dass** die wenigstens eine organische Verbindung ausgewählt ist aus der Gruppe bestehend aus Vasodilatatoren.

**14.** Verfahren nach einem der Ansprüche 1 bis 7, **dadurch gekennzeichnet, dass** die wenigstens eine organische Verbindung ausgewählt ist aus der Gruppe bestehend aus blutungshemmenden oder blutstillenden Mitteln.

**15.** Verfahren nach einem der Ansprüche 1 bis 7, **dadurch gekennzeichnet, dass** die wenigstens eine organische Verbindung ausgewählt ist aus der Gruppe bestehend aus nicht-steroidalen Entzündungshemmern.

**16.** Verfahren nach einem der Ansprüche 1 bis 7, **dadurch gekennzeichnet, dass** die wenigstens eine organische Verbindung ausgewählt ist aus der Gruppe bestehend aus antimikrobiellen Mitteln.

**17.** Verfahren nach einem der Ansprüche 1 bis 7, **dadurch gekennzeichnet, dass** die wenigstens eine organische Verbindung ausgewählt ist aus der Gruppe bestehend aus Glykosiden und Derivaten davon.

**18.** Verfahren nach einem der Ansprüche 1 bis 7, **dadurch gekennzeichnet, dass** die wenigstens eine organische Verbindung ausgewählt ist aus der Gruppe bestehend aus feuchtigkeitsspendenden und gewebeaufbauenden Mitteln.

**19.** Verfahren nach einem der vorangehenden Ansprüche, **dadurch gekennzeichnet, dass** das Hydrogel aus wenigstens einem Polysaccharid besteht, das ausgewählt ist aus der Gruppe bestehend aus Hyaluronsäure, Keratan, Heparin, Zellulose, Zellulosederivaten, Alginsäure, Xanthan, Carrageen, Chitosan, Chondroitin, Heparosan und deren biologisch akzeptablen Salzen, alleine oder als Mischung.

**20.** Verfahren nach Anspruch 19, **dadurch gekennzeichnet, dass** das Polysaccharid Hyaluronsäure ist.


**Claims**

**1.** A method for incorporating at least one organic compound within a hydrogel, wherein said incorporation is carried out by dialysis, said organic compound being solubilised in the dialysis solution at a concentration equal or greater than the target concentration of organic compound in the gel after incorporation, said target concentration being from 0,01 to 500 mg/g.

**2.** The method according to claim 1, wherein said dialysis solution is water or an aqueous solution which can further comprise mineral salts.

**3.** The method according to any one of the proceding claims, wherein said dialysis solution comprises sodium chloride.

**4.** The method according to any one of the proceding claims, wherein said dialysis solution further comprises a pH buffer.

**5.** The method according to any one of the proceding claims, wherein said dialysis is carried out using a membrane, the cutt-off threshold (MCWO) of which is from 1 to 200 kD ($1 \leq MCWO \leq 200$ kD).

**6.** The method according to any one of the proceding claims, wherein said dialysis is carried out in a closed environment.

**7.** The method according to any one of claims 1 to 5, wherein said dialysis is carried out continuously by implementation of a continous flow of dialysis solution through the hydrogel and the membrane.

**8.** The method according to any one of the proceding claims, wherein at least one organic compound is selected from the group consisting of the antioxidants.

9. The method according to any one of claims 1 to 7, wherein at least one organic compound is selected from the group consisting of the local anaesthetics.

10. The method according to any one of claims 1 to 7, wherein at least one organic compound is selected from the group consisting of the vitamines.

11. The method according to any one of claims 1 to 7, wherein at least one organic compound is selected from the group consisting of the amino acids.

12. The method according to any one of claims 1 to 7, wherein at least one organic compound is selected from the group consisting of the vasoconstrictors.

13. The method according to any one of claims 1 to 7, wherein at least one organic compound is selected from the group consisting of the vasodilators.

14. The method according to any one of claims 1 to 7, wherein at least one organic compound is selected from the group consisting of the antihemorrhagic or hemostatic agents.

15. The method according to any one of claims 1 to 7, wherein at least one organic compound is selected from the group consisting of nonsteroidal antiinflammatories.

16. The method according to any one of claims 1 to 7, wherein at least one organic compound is selected from the group consisting of the antimicrobial agents.

17. The method according to any one of claims 1 to 7, wherein at least one organic compound is selected from the group consisting of the glycosides and derivatives thereof.

18. The method according to any one of claims 1 to 7, wherein the at least one organic compound is selected from the group consisting of the hydrating agents or tissue regenerating agents.

19. The method according to any one of the proceding claims, wherein the hydrogel consists of at least one polysaccharide selected from the group consisting of hyaluronic acid, keratan, heparin, cellulose, cellulose derivatives, alginic acid, xanthan, carrageenan, chitosan, chondroitin, heparosan and the biologically acceptable salts thereof, alone or in mixture.

20. The method according to claim 19, wherein saidpolysaccharide is hyaluronic acid.

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2005067994 A
- WO 2010052430 A
- WO 2012104419 A
- WO 2007128923 A
- WO 2010015901 A
- WO 2017036597 A
- WO 2013185934 A

- WO 2000046253 A
- WO 2009071697 A
- WO 2004092222 A
- FR 2983483
- WO 2017162676 A
- WO 8600079 A

**Littérature non-brevet citée dans la description**

- **R.F. BOYER.** Modem experimental biochemistry. Addison-Wesley Publishing Co, 1986, 42-44